# EUROPÄISCHE PATENTSCHRIFT

(11) **EP 1 432 503 B1**
(45) Veröffentlichungstag und Bekanntmachung des Hinweises auf die Patenterteilung: **25.04.2007**
(21) Anmeldenummer: 02762205.9
(22) Anmeldetag: 01.10.2002
(51) Int. Cl.: B01F 13/00, B65D 81/32, A61C 5/06, A61M 5/28

(54) **MISCHKAPSEL SOWIE VERFAHREN ZUM AKTIVIEREN DER MISCHKAPSEL**
MIXING CAPSULE AND METHOD FOR ACTIVATING THE SAME
CAPSULE DE MELANGE ET SON PROCEDE D'ACTIVATION

(30) Priorität: 01.10.2001 CH 179601
(43) Veröffentlichungstag der Anmeldung: 30.06.2004
(73) Patentinhaber: Alfred Schmid AG Gossau, 9200 Gossau (CH)
(72) Erfinder: SCHMID, Daniel, CH-9200 Gossau (CH)
(74) Vertreter: Hasler, Erich
(86) Internationale Anmeldenummer: PCT/CH2002/000546
(87) Internationale Veröffentlichungsnummer: WO 2003/028871

(56) Entgegenhaltungen:
- EP-A- 0 245 788
- WO-A-00/45732
- DE-A- 4 232 062
- DE-C- 4 315 920
- GB-A- 1 326 289

## Beschreibung

Die Erfindung betrifft eine Mischkapsel für eine Zweikomponentenmischung sowie ein Verfahren zum Aktivieren einer wenigstens zwei Komponenten einer Mehrkomponentenmischung aufnehmenden Mischkapsel.

Die EP-A-0 245 788 offenbart eine Mischkapsel für eine Zweikomponentenmischung mit einem zylindrischen Gehäuse mit angeformter Ausspritzdüse und einem im zylindrischen Gehäuse axial beweglichen Kolben. Der Kolben besitzt am Umfang ein Gewinde, welches mit einem entsprechenden Innengewinde des zylindrischen Gehäuses zusammenwirken kann. Die Stirnseite des Kolbens hat eine zentrale Öffnung, durch welche Flüssigkeit in das Kolbeninnere fliessen kann. Ein an der Stirnseite des Kolbens angeordnetes Flüssigkeitskissen dient als Flüssigkeitsreservoir. Das Flüssigkeitskissen besitzt eine an der Stirnseite des Kolbens anliegende Membran, welche bei Überdruck im Bereich der Oeffnung berstet. Die Rückseite des Kolbens ist mit einem Stopfen verschlossen, und der Kolbeninnenraum dient der Aufnahme der pulverförmigen Komponente der Zweikomponentenmischung. Zum Mischen der Komponenten wird der Kolben soweit in das Gehäuse eingedreht, bis die Membran des Flüssigkeitskissen platzt und sich die Flüssigkeit in das Kolbeninnere ergiesst. Beim Zerplatzen der Membran franst diese aus, und es können Teile der Membran in den Mischraum gelangen.

Eine zur EP-A-0 245 788 ähnliche Mischkapsel offenbart die US 5,026,283. Auch bei dieser Kapsel wird die die Flüssigkeitspackung vom Mischraum trennende Membran unter Anwendung eines Überdrucks zerplatzt. Demzufolge können auch bei dieser Kapsel lose Membranschnitzel in den Mischraum gelangen. Um die Mischung auspressen zu können, muss die vordere Membran der Flüssigkeitspackung zuerst mit einem an einem weiteren Kolben angeordneten Stift von der Rückseite her durchstossen werden.

Charakteristisch bei den oben beschriebenen Kapseln ist, dass beim Aktivieren Membranteile in den Mischraum und somit in die Mischung gelangen können. Nachteilig ist auch, dass beim Aktivieren der Kapsel ein Überdruck in der Mischkammer entsteht. Der Überdruck kommt dadurch zustande, dass ein zusätzliches Volumen, nämlich das der Flüssigkeit, in den Mischraum gepresst wird. Ein Überdruck beim Mischen der Komponenten bewirkt jedoch, dass Gas in die Mischung gepresst wird und sich beim Ausdrücken unerwünschte Gasblasen bilden können.

Aufgabe der vorliegenden Erfindung ist es, eine Mischkapsel bereitzustellen, bei welcher beim Aktivieren keine Membranschnitzel in den Mischraum gelangen können. Weiteres Ziel ist, eine Kapsel zu schaffen, die mit geringem Kraftaufwand aktiviert werden kann. Noch ein Ziel ist es, eine Kapsel vorzuschlagen, bei welcher die Gefahr der Blasenbildung gering ist. Des weiteren soll eine Kapsel bereitgestellt werden, welche kostengünstig herstell- und beladbar ist.

Erfindungsgemäss ist eine Kapsel dadurch gekennzeichnet, dass im einen Mischraum definierenden Hohlraum zwischen den Stirnseiten des Behälterteils und des Kolbens ein beweglicher Verdrängungskörper mit einer zum Flüssigkeitsbehältnis komplementären Form vorgesehen ist. Diese Kapsel hat den Vorteil, dass mit dem Verdrängungskörper die Membran in Richtung Flüssigkeitsraum durchstossen wird und infolgedessen keine Membranteile in den Mischraum gelangen. Weiter ist nur eine geringe Kraft erforderlich, um die Membran zu zerstören. Entsprechend sind keine Gewinde beim Behälterteil und Kolben vorzusehen. Ein weiterer Vorteil ist, dass beim Aktivierungsvorgang, d.h. wenn die im Mischraum und im Flüssigkeitsbehältnis gelagerten Komponenten zusammengebracht werden, das Volumen der Mischkammer gleich bleibt und kein Überdruck entsteht. Der erfindungsgemässe Verdrängungskörper hat neben der Aktivierungsfunktion auch die Funktion eines Verdrängungsorgans, welches beim Aktivieren die flüssige Komponente aus dem Flüssigkeitsbehältnis verdrängt. Zweckmässigerweise passt der Verdrängungskörper im wesentlichen in die Öffnung an der Stirnseite des Kolbens, d.h. deren Aussen- resp. Innendurchmesser sind im wesentlichen gleich gross. Dies hat den Vorteil, dass bei eingeschobenem Verdrängungskörper die Membran durch den Verdrängungskörper gehalten ist und somit keine Membranteile in den Mischraum gelangen. Vorzugsweise ist der Verdrängungskörper mittels eines die Ausspritzdüse im nicht-aktivierten Zustand verschliessenden Aktivierungsstifts verschiebbar.

Vorteilhaft hat der Verdrängungskörper eine zum Flüssigkeitsbehältnis komplementäre Form. Dadurch kann der Verdrängungskörper die im Behältnis vorhandene Flüssigkeit in den Mischraum verdrängen. Weiterer Vorteil ist, dass die Membran durch das Aktivierungs- oder Verdrängungsteil im Flüssigkeitsbehältnis fixiert ist. Zweckmässigerweise ist der Verdrängungskörper im aktivierten Zustand der Kapsel im wesentlichen bündig mit der Stirnseite des Kolbens im Flüssigkeitsbehältnis aufgenommen. Dies hat den Vorteil, dass die fertige Mischung mit dem Kolben praktisch vollständig aus der Kapsel ausgepresst werden kann.

Obwohl das Flüssigkeitsbehältnis grundsätzlich eine aus einer Folie gebildete Packung sein kann, ist die Flüssigkeit vorzugsweise in einem am Kolben ausgebildeten Flüssigkeitsbehältnis aufgenommen. Gemäss einer besonders bevorzugten Ausführungsform ist der Kolben selbst als Flüssigkeitsbehältnis ausgebildet resp. an diesem angeformt. Dies ist eine einfache Konstruktion und entsprechend kostengünstig herstellbar. Vorteilhaft ist eine die Durchgangsöffnung überdeckende Membran vorne auf die Stirnseite des Kolbens aufgebracht resp. aufbringbar. Da die Kolbenwand eine bestimmte Wandstärke hat, kann die Folie gut auf die Stirnseite aufgeschweisst werden. Dabei besteht praktisch keine Gefahr, dass beim Aufschweissen Flüssigkeit verdampfen könnte. Zweckmässigerweise ist vorne am Kolbenmantel wenigstens ein Dichtring angeformt. Dieser sorgt für die Abdichtung der durch die Stirnseiten des Behälterteils und des Kolbens definierten Mischkammer.

Vorteilhaft ist das Vorderteil des Verdrängungskörpers als Spitze ausgebildet. Dadurch ist es möglich, die das Flüssigkeitsbehältnis verschliessende Folie mit geringem Kraftaufwand zu durchstossen. Gemäss einer bevorzugten Ausführungsform ist im Mantel des Verdrängungskörpers ein vorzugsweise in axialer Richtung verlaufender Überstromkanal resp. eine Rinne vorgesehen. Der Überstromkanal kann beim Einführen des Verdrängungskörpers einen Druckaufbau im Flüssigkeitsbehältnis verhindern, da die Flüssigkeit gut abfliessen kann. Weiter kann der Überstromkanal so dimensioniert sein, dass auch viskose oder zähflüssige Medien aus dem Flüssigkeitsbehältnis ausbringbar sind.

Grundsätzlich kann der Aktivierungsstift direkt am Verdrängungskörper angeformt sein. In diesem Fall kann der Verdrängungskörper zusammen mit dem Aktivierungsstift von hinten in das Behälterteil eingesetzt werden. Ein Sollbruchstelle am Boden des Verdrängungskörpers erlaubt es, den Aktivierungsstift nach dem Mischvorgang, z.B. durch Drehen, vom Aktivierungsteil zu trennen und zurückzuziehen. Gemäss einer bevorzugten Ausführungsform sind jedoch Aktivierungsteil und Aktivierungsstift als separate Teile ausgebildet. Bei dieser Ausführungsform ist zweckmässigerweise am Boden des Verdrängungskörpers eine als Führung für den Aktivierungsstift dienende Vertiefung vorgesehen. Der Verdrängungskörper kann auf den Aktivierungsstift, welcher vorgängig in die Ausspritzdüse eingeführt wird, aufgesteckt werden. Zweckmässigerweise ist der Verdrängungskörper ein Füllkörper mit einem flachen Boden. Der Füllkörper kann aus Kunststoff sein und eine zum Flüssigkeitsbehältnis komplementäre Form haben. Vorteilhaft passt der Füllkörper ungefähr bündig mit der Stirnseite des Kolbens in das Flüssigkeitsbehältnis.

Gemäss einer besonders bevorzugten Ausführungsform hat der Aktivierungsstift eine solche Länge, dass der Kolben der gefüllten Mischkapsel von einer Füllstellung um eine bestimmte Distanz in eine Mischstellung zurückschiebbar ist. Durch die Vergrösserung des Volumens resultiert ein Unterdruck in der Mischkammer. Dies hat den Vorteil, dass beim Mischen eine Entgasung der Mischung stattfindet. Somit kommt es beim Ausdrücken der Mischung zu keiner Blasenbildung.

Gegenstand der vorliegenden Erfindung ist auch ein Verfahren zum Mischen einer wenigstens zwei Komponenten einer Mehrkomponentenmischung aufnehmenden Mischkapsel, dadurch gekennzeichnet, dass die Membran zerstört wird, indem ein im Mischraum vorgesehener, beweglicher Verdrängungskörper in das Flüssigkeitsbehältnis gestossen wird. Dadurch, dass die Membran in das Flüssigkeitsbehältnis gestossen wird, besteht keine Gefahr, dass Teile der Membran in den Mischraum gelangen. Vorteilhaft drückt der Verdrängungskörper mit einer zum Flüssigkeitsbehältnis komplementären Form die Flüssigkeit aus dem Flüssigkeitsbehältnis in den Mischraum. Bevorzugt wird der Mischraum vor dem Mischen vergrössert, sodass der Mischvorgang beim Unterdruck stattfindet. Dies hat den Vorteil, dass beim Mischen eine Entgasung der Mischung stattfinden kann.

Die Erfindung wird nachfolgend beispielhaft unter Bezugnahme auf die Figuren erläutert. Es zeigt
- Figur 1:: eine erfindungsgemässe Mischkapsel im Längsschnitt und in der Füllstellung (Ausgangszustand);
- Figur 2:: die Kapsel von Figur 1 im aktivierten Zustand (Mischstellung);
- Figur 3:: die Kapsel von Figur 2, nach dem der Aktivierungsstift zurückgezogen ist (Auspressstellung);
- Figur 4:: ein konischers Verdrängungskörper;
- Fig. 5:: eine zweite Ausführungsform einer Mischkapsel mit einem Verdrängungskörper, welches am Behälterboden fixierbar ist; und
- Fig. 6:: in perspektivischer Ansicht eine Ausführungsform des Verdrängungskörpers 33 mit ringförmigen Vorsprüngen am Boden.

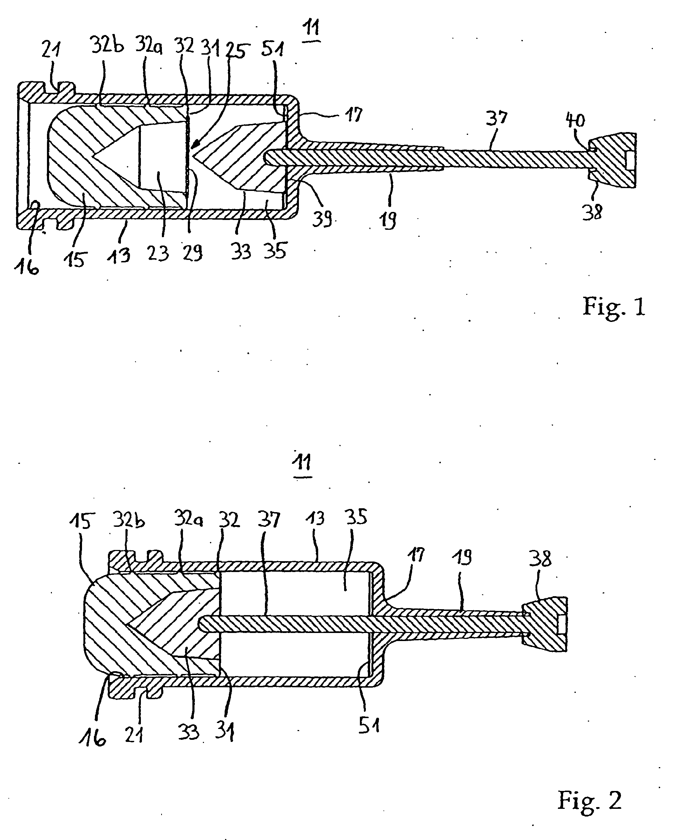

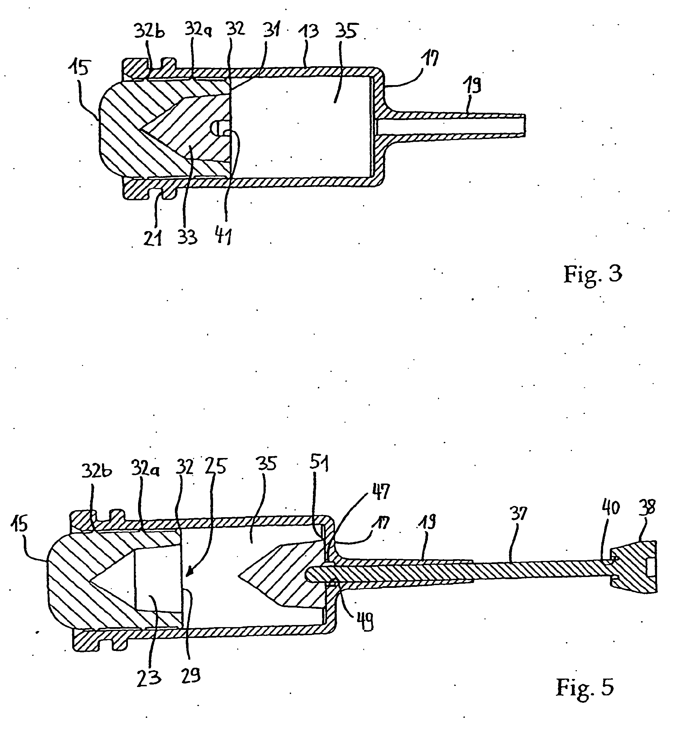

Die Figuren 1 bis 4 zeigen eine erfindungsgemässe Mischkapsel 11 mit einem äusseren Behälterteil 13 und einem im Behälterteil 13 aufgenommenen Kolben 15. Der Kolben 15 ist im Behälterteil 13 axial verschiebbar und befindet sich in Figur 1 in der Ausgangsposition oder Füllstellung und in Figur 2 in der Mischstellung (aktivierter Zustand der Kapsel). Das Behälterteil 13 ist zylindrisch und hat eine Öffnung 16 zum Einführen des Kolbens 15 und eine Stirnseite 17, an welcher eine Ausspritzdüse 19 angeformt ist. Am hinteren Ende des Behälterteils ist aussen eine Ringnut 21 vorgesehen. Die Ringnut 21 dient der Aufnahme einer Backe eines in der Branche bekannten Auspresswerkzeugs.

Am Kolben 15 ist ein Flüssigkeitsbehältnis 23 vorgesehen. Gemäss der gezeigten vorteilhaften Ausführungsform hat der Kolben 15 die Gestalt eines Bechers mit einer Öffnung 25 und einem Innenraum 27. Der Innenraum 27 dient der Aufnahme der flüssigen Komponente eines Zweikomponentenharzes und dient als formstabiles Flüssigkeitsbehältnis 23. Im nicht-aktivierten Zustand ist die Öffnung 25 mit einer Folie oder Membran 29 verschlossen. Die Folie ist in bekannter Art auf die Stirnseite 31 des Kolbens 15 aufgeschweisst. Zur Abdichtung des Kolbens 15 gegen das Behälterteil 13 sind am Kolbenmantel eine oder mehrere Ringdichtungen 32 angeformt. Die erste Dichtung 32 befindet sich am vordersten Kolbenrand. Zwei weitere Dichtungen 32a, 32b befinden in Abstand zur ersten Dichtung 32.

Bei zusammengesetzter Mischkapsel ist zwischen dem Kolben 15 und der Stirnseite des Behältnisses 13 ein Mischraum 35 definiert. Ein Verdrängungskörper 33 ist in den Mischraum 35 zwischen den Stirnseiten des Kolbens 15 und des Behälterteils 13 eingelegt. Der Verdrängungskörper 33 ist ein Aktivierungsteil, welches eine zum Innenraum 27 komplementäre Gestalt hat. Der Verdrängungskörper 33 ist mittels eines Aktivierungsstiftes 37, welcher in der Ausspritzdüse 19 aufgenommen ist, im Behälterteil 13 axial verschiebbar. Die Länge des Aktivierungsstifts 37 ist wenigstens so lang, dass der Verdrängungskörper 33 vollständig in das Flüssigkeitsbehältnis 23 eingeschoben werden kann. Der Aktivierungsstift 37 hat einen Kopf 38, welcher als Anschlag dient. Der Kopf 38 hat eine Hinterschneidung 40, in welcher der vordere Rand der Ausspritzdüse bei vollständig eingeschobenem Aktivierungsstift 37 aufgenommen ist.

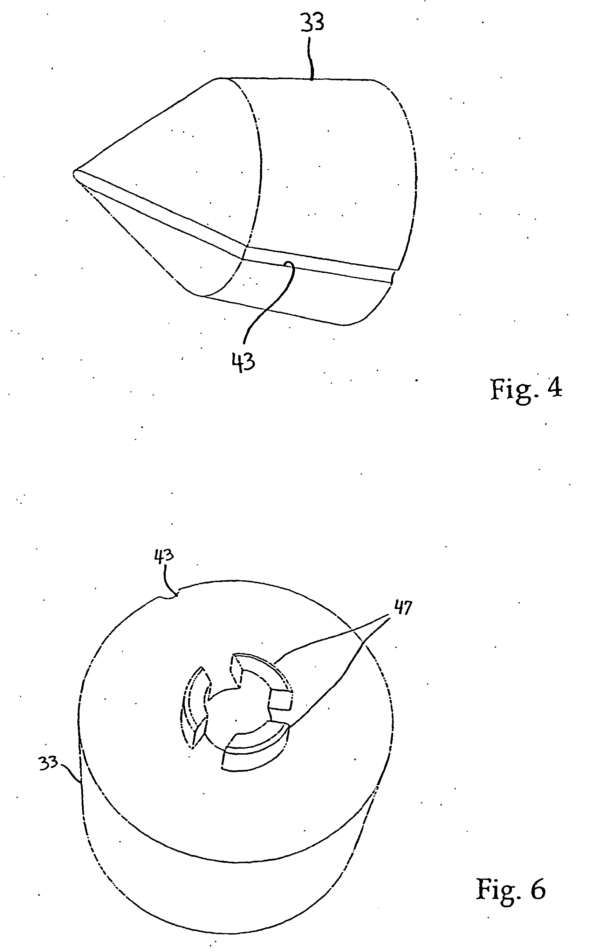

Der Verdrängungskörper 33 besitzt am Boden 39 eine runde Vertiefung 41. Die Vertiefung 41 dient der Aufnahme des Vorderteils des Aktivierungsstifts 37. Vorteilhaft sind. Vorderteil des Aktivierungsstifts 37 und Vertiefung 41 so geschaffen, dass ein Reibschluss realisiert ist. Dadurch ist der Verdrängungskörper 33 im Mischraum der nicht-aktivierten Kapsel 11 fixiert. Denkbar ist jedoch auch, dass der Verdrängungskörper 33 mit radial abstehenden Armen ausgestattet ist, sodass er im Behälterteil 13 geführt ist. Um einen ungehinderten Fluss der im Flüssigkeitsbehältnis vorhandenen Flüssigkeit in den Mischraum 35 zu gewährleisten, ist im Mantel des Verdrängungskörpers ein Überstromkanal 43 vorgesehen (Fig. 4).

Eine modifizierte Ausführungsform des Verdrängungskörpers 33 sieht vor, dass am Boden 45 desselben Mittel vorgesehen sind, um der Verdrängungskörper 33 am Behälterteil 13 lösbar zu fixieren. Diese Mittel können - wie in den Fig.5 und 6 gezeigt - ringförmige Vorsprünge 47 sein, welche in einer Ringnut 49 am Behälterboden 51 einrasten können. Die Ringnut 49 ist an der Öffnung der Ausspritzdüse 19 vorgesehen. Die gezeigte Ausführungsform hat den Vorteil, dass beim Befüllen der Kapsel der Verdrängungskörper zuerst in das Behälterteil 13 eingesetzt werden kann und dass dann ein ungewolltes Herausfallen oder Verrutschen durch die Reibschlussverbindung zwischen Ringnut 49 und Vorsprüngen 47 verhindert ist.

Die erfindungsgemässe Mischkapsel wird wie folgt vorbereitet und eingesetzt: Das Aktivierungs- und Verdrängungsteil 33 wird zunächst in das Behälterteil 13 eingelegt und der Aktivierungsstift durch die Ausspritzdüse 19 soweit eingeführt, bis das Vorderteil des Aktivierungsstift 37 in der Vertiefung 41 aufgenommen ist Anschliessend wird die eine Kunstharzkomponente (pulverförmig oder flüssig) in das senkrecht ausgerichtete Behälterteil 13 eingefüllt.

Der Kolben 15, welcher gleichzeitig als Flüssigkeitsbehältnis 23 dient, wird in einer separaten Operation mit der gewünschten Flüssigkeit gefüllt und dicht verschweisst. Diese Vorgänge können automatisiert ablaufen. Die Befüllung des Kolbens 15 durch die Öffnung 25 und die Verschweissung der Folie mit dem Kolbenrand mittels eines heissen Stempels sind von oben einfach zu bewerkstelligen.

Anschliessend wird der Kolben in das Behälterteil 13 eingesetzt. Um einen Druckaufbau in der Mischkammer zu verhindern, kann zwischen Behälterwand und Kolben 15 ein Draht eingelegt sein, um beim Einführen des Kolbens Luft aus dem Mischkammer an die Umgebung abzulassen. Danach kann der Draht wieder entfernt werden. Die Position des Kolbens 15 im Behälterteil 13 ist vorzugsweise so gewählt dass der Kolben 15 der gefüllten Mischkapsel von einer Ausgangsstellung (Fig. 1) eine bestimmte Distanz nach hinten (aussen) in eine Mischstellung (Fig. 2) geschoben werden kann. Infolge des vergrösserten Volumens in der Mischstellung herrscht ein Unterdruck in der Mischkammer. Dies kann zu einer Entgasung der Mischung während des Mischvorgangs genutzt werden. Zum Mischen wird die Kapsel in einen bekannten Schüttelapparat eingesetzt. Dieser Schüttelapparat besitzt zwei in Abstand voneinander angeordnete und gegeneinander vorgespannte Haltegabeln. Zum Mischen wird die Kapsel zwischen den Gabeln eingespannt.

Zur Aktivierung der Kapsel 11 wird der Aktivierungskörper 33 mit Hilfe des Aktivierungsstifts 37 zunächst in das Flüssigkeitsbehältnis 23 gestossen, und sodann der ganze Kolben 15 in die Mischstellung verschoben. Dabei durchstösst der spitze Verdrängungskörper 33 die Folie 29 in der Mitte und presst die im Flüssigkeitsbehältnis 23 vorhandene flüssige Kunstharzkomponente in den Mischraum 35. In der dann eingenommenen Mischstellung wird die Kapsel 11 in einem bekannten Schüttelapparat während einer bestimmten Zeitspanne geschüttelt. Der Aktivierungsstift 37 wird danach herausgezogen, und die fertige, viskose Kunstharzmischung mit einer bekannten Auspresspistole ausgedrückt.

Eine Mischkapsel für eine Zweikomponentenmischung besitzt einen vorzugsweise zylindrischen Behälterteil 13 mit einer an der Stirnseite angeformten Ausspritzdüse 19. Ein Kolben ist im Behälterteil axial verschiebbar geführt. In der Stirnseite des Kolbens ist eine Öffnung vorgesehen, an welche ein Flüssigkeitsbehältnis mit einer berstbaren Wand oder Membran anschliesst. Im nicht-aktivierten Zustand der Kapsel verschliesst die Membran die Öffnung. Der Hohlraum zwischen den Stirnseiten des Behälterteils und des Kolbens bildet einen Mischraum. Im Mischraum ist ein bewegliches Aktivierungsteil vorgesehen. Der Verdrängungskörper ist mittels eines die Ausspritzdüse im nicht-aktivierten Zustand verschliessenden Aktivierungsstifts verschiebbar. Im aktivierten Zustand der Kapsel ist der Verdrängungskörper im wesentlichen vollständig im Flüssigkeitsbehältnis aufgenommen. Behälterteil 13 und Kolben 15 sind aus einem geeigneten Kunststoff hergestellt.

Die erfindungsgemässe Mischkapsel ist kostengünstig herstell- und befüllbar. Sie hat den den Vorteil, dass keine Membranschnitzel in den Mischraum gelangen können. Ausserdem wird immer die gesamte Flüssigkeitsmenge in den Mischraum gepresst, sodass eine gleichbleibende Qualität der Mischungen erreicht wird. Da keine Gewinde oder Hinterschneidungen vorhanden sind, besteht auch keine Gefahr, dass Flüssigkeit an diesen Stellen zurückbleibt. Der stirnseitige Rand des Kolbens kann die Füllöffnung bilden. Auf diesen können Folien aufgrund der genügend breiten Auflagefläche problemlos aufgeschweisst werden. Der als Flüssigkeitsbehältns dienende Kolben kann deshalb auch mit leicht flüchtigen Komponenten gefüllt werden.

### Legende:

- 11: Mischkapsel
- 13: Behälterteil
- 15: Kolben
- 16: Öffnung im Behälterteil 13 zum Einführen des Kolbens 15
- 17: Stirnseite des Behälterteils
- 19: Ausspritzdüse
- 21: Ringnut
- 23: Flüssigkeitsbehältnis
- 25: Öffnung (Durchgangsöffnung)
- 27: Innenraum
- 29: Folie oder Membran
- 31: Stirnseite des Kolbens
- 32: Dichtring oder Dichtwulst
- 32a,32b: zweiter und dritter Dichtring oder Dichtwulst
- 33: Verdrängungskörper
- 35: Mischraum
- 37: Aktivierungsstiftes
- 38: Kopf des Aktivierungsstiftes
- 39: Boden des Verdrängungskörpers
- 40: Hinterschneidung am Kopf 38
- 41: Vertiefung
- 43: Überstromkanal
- 45: Boden des Verdrängungskörpers 33
- 47: Vorsprünge, z.B. ringförmige
- 49: Ringnut 49
- 51: Behälterboden

## Patentansprüche

1. Mischkapsel für eine Zweikomponentenmischung mit einem vorzugsweise zylindrischen Behälterteil (13) mit einer Öffnung (16) und einer gegenüberliegenden Stirnseite (17), an welcher eine Ausspritzdüse (19) angeformt ist, einem im Behälterteil (13) axial beweglichen Kolben (15), einer Öffnung (25) in der Stirnseite (31) des Kolbens (15), einem am Kolben (15) angeordneten Behältnis (23) zur Aufnahme einer Flüssigkeit mit einer berstbaren Wand oder Membran (29), welche die Öffnung (25) im nicht-aktivierten Zustand der Kapsel (11) verschliesst, **dadurch gekennzeichnet, dass** im Hohlraum (35) zwischen den Stirnseiten des Behälterteils (13) und des Kolbens (15) ein beweglicher Verdrängungskörper (33) mit einer zum Flüssigkeitsbehältnis (23) komplementären Form vorgesehen ist.

2. Mischkapsel nach Anspruch 1, **dadurch gekennzeichnet, dass** der Verdrängungskörper (33) im wesentlichen in die Öffnung (25) passt.

3. der Verdrängungskörper Mischkapsel nach Anspruch 1 oder 2, **dadurch gekennzeichnet dass** der Verdrängungskörper (33) im aktivierten Zustand der Kapsel (11) im wesentlichen bündig mit der Stirnseite (31) des Kolbens (15) im Flüssigkeitsbehältnis (23) aufgenommen ist.

4. Mischkapsel nach einem der Ansprüche 1 bis 3, **dadurch gekennzeichnet dass** der Kolben (15) das Flüssigkeitsbehältnis (23) bildet.

5. Mischkapsel nach einem der Ansprüche 1 bis 4, **dadurch gekennzeichnet, dass** eine die Öffnung (25) überdeckende Membran (29) vorne auf die Stirnseite (31) des Kolbens (15) aufgebracht ist.

6. Mischkapsel nach Anspruch 5, **dadurch gekennzeichnet, dass** die Membran (29) auf den Kolben (15) aufgeschweisst ist.

7. Mischkapsel nach einem der Ansprüche 1 bis 6, **dadurch gekennzeichnet dass** am Kolbenmantel wenigstens ein Dichtring oder Dichtwulst (32) angeformt ist.

8. Mischkapsel nach einem der Ansprüche 1 bis 7, **dadurch gekennzeichnet dass** das Vorderteil Verdrängungskörpers (33) spitz zulaufend ist.

9. Mischkapsel nach einem der Ansprüche 1 bis 8, **dadurch gekennzeichnet, dass** im Mantel des Verdrängungskörpers (33) ein in axialer Richtung verlaufender Überstromkanal (43) vorgesehen ist.

10. Mischkapsel nach einem der Ansprüche 1 bis 9, **dadurch gekennzeichnet dass** der Verdrängungskörper (33) mittels eines die Ausspritzdüse (19) im nicht-aktivierten Zustand verschliessenden Aktivierungsstifts (37) verschiebbar ist.

11. Mischkapsel nach Anspruch 10, **dadurch gekennzeichnet, dass** der Verdrängungskörper (33) integral mit dem Aktivierungsstift (37) ist.

12. Mischkapsel nach Anspruch 10 oder 11, **dadurch gekennzeichnet, dass** zwischen Verdrängungskörper (33) und Aktivierungsstift (37) eine Sollbruchstelle vorgesehen ist.

13. Mischkapsel nach einem der Ansprüche 1 bis 12, **dadurch gekennzeichnet, dass** am Boden (45) des Verdrängungskörpers (33) eine als Führung für den Aktivierungsstift dienende Vertiefung (41) vorgesehen ist.

14. Mischkapsel nach einem der Ansprüche 1 bis 13 **dadurch gekennzeichnet, dass** der Verdrängungskörper (33) ein Füllkörper mit einem flachen Boden ist.

15. Mischkapsel nach einem der Ansprüche 11 bis 14, **dadurch gekennzeichnet, dass** der Aktivierungsstift (37) eine solche Länge hat, dass der Kolben (15) der gefüllten Mischkapsel von einer Ausgangsstellung um eine bestimmte Distanz in eine Mischstellung zurückschiebbar ist.

16. Verfahren zum Aktivieren einer wenigstens zwei Komponenten einer Mehrkomponentenmischung aufnehmenden Mischkapsel (11) mit einem vorzugsweiese zylindrischen Behälterteil mit einer Öffnung (16) und an einer Stirnseite (17), an welcher eine Ausspritzdüse (19) angeformt ist, einem im Gehäuse (11) axial beweglichen Kolben (15), einer Öffnung (25) in der Stirnseite (31) des Kolbens (15), einem im oder am Kolben (15) angeordneten Behältnis (23) zur Aufnahme einer Flüssigkeit mit einer berstbaren Wand oder Membran (29), welche die Öffnung (25) im nicht-aktivierten Zustand der Kapsel verschliesst, bei welchem Verfahren eine Membran oder Folie, welche den eine pulverförmige Komponente enthaltenden Mischraum von einem Flüssigkeitsbehältnis trennt, zerstört und der Inhalt des Flüssigkeitsbehältnis in den Mischraum gepresst wird, **dadurch gekennzeichnet, dass** die Membran (29) zerstört wird, indem ein beweglicher Verdrängungskörper (33) mit einer zum Flüssigkeitsbehältnis (23) komplementären Form in das Flüssigkeitsbehältnis (23) gestossen wird.

17. Verfahren nach Anspruch 16, **dadurch gekennzeichnet, dass** der Verdrängungskörper (33) die Flüssigkeit aus dem Flüssigkeitsbehältnis (27) in den Mischraum (35) drückt.

18. Verfahren nach Anspruch 16 oder 17, **dadurch gekennzeichnet, dass** der Mischraum (35) vor dem Mischen vergrössert wird, sodass der Mischvorgang bei Unterdruck stattfindet.

## Claims

1. A mixing capsule for a two-component mixture with a preferably cylindrical container part (13) with an opening (16) and a front face (17) situated opposite it, on which front face there is formed a discharge nozzle (19), a piston (15) that can be axially displaced inside the container part (13), an opening (25) in the front face (31) of the piston (15), a liquid receptacle (23) arranged on the piston (15) with a burstable wall or membrane (29) that closes the opening (25) in the non-activated condition of the capsule (11), **characterized in that** in the hollow (35) between the front faces of the container part (13) and the piston (15) there is provided a displaceable activation part (33) having a shape that is complementary to the liquid receptacle (23).

2. A mixing capsule in accordance with Claim 1, **characterized in that** the activation part (33) fits essentially into the opening (25).

3. A mixing capsule in accordance with Claim 1 or 2, **characterized in that** in the activated condition of the capsule the activation part (33) is accommodated in the liquid receptacle (23) substantially flush with the front face of the piston (15).

4. A mixing capsule in accordance with any one of Claims 1 to 3, **characterized in that** the piston (15) constitutes the liquid receptacle (23).

5. A mixing capsule in accordance with any one of Claims 1 to 4, **characterized in that** a membrane (29) covering the opening (25) is attached to the front face of the piston (15).

6. A mixing capsule in accordance with Claim 5, **characterized in that** the membrane (29) is welded onto the piston (15).

7. A mixing capsule in accordance with any one of Claims 1 to 8, **characterized in that** at least one gasket ring or sealing bulge (32) is formed on the piston jacket.

8. A mixing capsule in accordance with any one of Claims 1 to 7, **characterized in that** the front part of the activation part (33) is designed as tapering into a tip.

9. A mixing capsule in accordance with any one of Claims 1 to 8, **characterized in that** an overflow channel (43) extending in the axial direction is provided on the jacket of the activation part (33).

10. A mixing capsule in accordance with any one of Claims 1 to 9, **characterized in that** the activation part (33) can be displaced by means of activation pin (37) that closes the discharge nozzle (19) in the non-activated condition.

11. A mixing capsule in accordance with Claim 10, **characterized in that** the activation part (33) is integral with the activation pin (37).

12. A mixing capsule in accordance with Claim 10 or Claim 11, **characterized in that** a predetermined breaking point is provided between the activation part (33) and the activation pin (37).

13. A mixing capsule in accordance with any one of Claims 1 to 12, **characterized in that** a recess (41) that serves as a guide for the activation pin is provided on the bottom of the activation part (33).

14. A mixing capsule in accordance with any one of Claims 1 to 13, **characterized in that** the activation part (33) is a filling body with a flat bottom.

15. A mixing capsule in accordance with any one of Claims 11 to 14, **characterized in that** the length of the activation pin (37) is such that the piston (15) of the filled mixing capsule can be pushed back over a certain distance from an initial position to a mixing position.

16. A method of activating a mixing capsule (11) for receiving at least two components of a multi-component mixture with a preferably cylindrical containing part with an opening (16) and a front face (17) situated opposite it, on which there is formed a discharge nozzle (19), a piston (15) that can be axially displaced inside the housing (11), an opening (25) in the front face (31) of the piston (15), a liquid receptacle (23) arranged in or on the piston (15) with a burstable wall or membrane (29) that closes the opening (25) in the non-activated condition of the capsule, in which method a membrane or foil that separates the mixing chamber containing a pulverous component from the liquid receptacle is destroyed and the content of the liquid receptacle is pressed into the mixing chamber, **characterized in that** the membrane is destroyed by pushing a displaceable activation part (33) having a shape that is complementary to the liquid receptacle (23) into the liquid receptacle (23).

17. A method in accordance with Claim 16, **characterized in that** the activation part (33) presses the liquid from the liquid receptacle (23) into the mixing chamber (35).

18. A method in accordance with Claim 16 or Claim 17, **characterized in that** mixing chamber (35) is enlarged before the mixing, so that the mixing process takes place in low-pressure conditions.

## Revendications

1. Capsule de mélange pour un mélange à deux composants avec une partie réservoir de préférence cylindrique (13) avec une ouverture (16) et un côté frontal opposé (17) sur lequel une tuyère de pulvérisation (19) est moulée, un piston (15) mobile axialement dans la partie réservoir (13), une ouverture (25) dans le côté frontal (31) du piston (15), un réservoir (23) placé sur le piston (15) pour recevoir un liquide avec une paroi ou membrane éclatable (29) qui ferme l'ouverture (25), lorsque la capsule est à l'état non activé, **caractérisée en ce qu'**un corps déplaceur (33) mobile avec une forme complémentaire de celle du réservoir de liquide (23) est prévu dans l'espace creux (35) entre les côtés frontaux de la partie réservoir (13) et du piston.

2. Capsule de mélange selon la revendication 1, **caractérisée en ce que** le corps déplaceur (33) est substantiellement ajusté dans l'ouverture (25).

3. Capsule de mélange selon la revendication 1 ou 2, **caractérisée en ce que** le corps déplaceur (33) est logé dans le réservoir de liquide (23), lorsque la capsule (11) est à l'état activé, en étant essentiellement de niveau avec le côté frontal (31) du piston (15).

4. Capsule de mélange selon l'une des revendications 1 à 3, **caractérisée en ce que** le piston (15) forme le réservoir de liquide (23).

5. Capsule de mélange selon l'une des revendications 1 à 4, **caractérisée en ce qu'**une membrane (29) qui recouvre l'ouverture (25) est posée devant sur le côté frontal (31) du piston (15).

6. Capsule de mélange selon la revendication 5, **caractérisée en ce que** la membrane (29) est soudée sur le piston (15).

7. Capsule de mélange selon l'une des revendications 1 à 6, **caractérisée en ce qu'**au moins un anneau d'étanchéité ou bourrelet d'étanchéité (32) est moulé sur l'enveloppe du piston.

8. Capsule de mélange selon l'une des revendications 1 à 7, **caractérisée en ce que** la partie antérieure du corps déplaceur (33) est effilée.

9. Capsule de mélange selon l'une des revendications 1 à 8, **caractérisée en ce qu'**un conduit de trop-plein (43) qui va dans le sens axial est prévu dans l'enveloppe du corps déplaceur (33).

10. Capsule de mélange selon l'une des revendications 1 à 9, **caractérisée en ce que** le corps déplaceur (33) est déplaçable à l'aide d'une broche d'activation (37) qui ferme la tuyère de pulvérisation (19) à l'état non activé.

11. Capsule de mélange selon la revendication 10, **caractérisée en ce que** le corps déplaceur (33) fait partie intégrale de la broche d'activation (37).

12. Capsule de mélange selon la revendication 10 ou 11, **caractérisée en ce qu'**un point destiné à la rupture est prévu entre le corps déplaceur (33) et la broche d'activation (37).

13. Capsule de mélange selon l'une des revendications 1 à 12, **caractérisée en ce qu'**un évidement (41) qui sert de guidage à la broche d'activation est prévu au fond (45) du corps déplaceur (33).

14. Capsule de mélange selon l'une des revendications 1 à 13, **caractérisée en ce que** le corps déplaceur (33) est un corps de remplissage avec un fond plat.

15. Capsule de mélange selon l'une des revendications 11 à 14, **caractérisée en ce que** la broche d'activation (37) a une longueur telle que le piston (15) de la capsule de mélange remplie peut être repoussée d'une position de départ d'une certaine distance à une position de mélange.

16. Procédé pour activer une capsule de mélange (11) qui reçoit au moins deux composants d'un mélange à deux composants avec une partie réservoir de préférence cylindrique (13) avec une ouverture (16) et un côté frontal opposé (17) sur lequel une tuyère de pulvérisation (19) est moulée, un piston (15) mobile axialement dans le boîtier (11), une ouverture (25) dans le côté frontal (31) du piston (15), un réservoir (23) placé dans ou sur le piston (15) pour recevoir un liquide avec une paroi ou membrane éclatable (29) qui ferme l'ouverture (25), lorsque la capsule est à l'état non activé, procédé pour lequel une membrane ou feuille qui sépare l'espace de mélange qui contient un composant poudreux d'un réservoir de liquide est détruite et le contenu du réservoir de liquide est pressé dans l'espace de mélange, **caractérisé en ce que** la membrane (29) est détruite tandis qu'un corps déplaceur mobile (33) avec une forme complémentaire de celle du réservoir de liquide (23) est poussé dans le réservoir de liquide (23).

17. Procédé selon la revendication 16, **caractérisé en ce que** le corps déplaceur (33) pousse le liquide du réservoir de liquide (27) dans l'espace de mélange (35).

18. Procédé selon la revendication 16 ou 17, **caractérisé en ce que** l'espace de mélange (35) est agrandi avant le mélange si bien que l'opération de mélange se fait en dépression.
